# EUROPEAN PATENT SPECIFICATION

(11) **EP 3 542 290 B1**
(45) Date of publication and mention of the grant of the patent: **03.01.2024**
(21) Application number: 17804385.7
(22) Date of filing: 09.11.2017
(51) Int. Cl.: G16B 5/00, G16B 5/20, G16H 50/50

(54) **VALIDATION OF INFERRED ANTICANCER PATHWAYS**
VALIDIERUNG VON ABGELEITETEN ANTIKREBSSIGNALWEGEN
VALIDATION DE VOIES ANTICANCÉREUSES INFÉRÉES

(30) Priority: 17.11.2016 US 201662423759 P
(43) Date of publication of application: 25.09.2019
(73) Proprietor: NantBio, Inc., Culver City, California 90232 (US); Nant Holdings IP, LLC, Culver City, CA 90232 (US)
(72) Inventor: NIAZI, Kayvan, Culver City, CA 90232 (US); RABIZADEH, Shahrooz, Culver City, CA 90232 (US); WITCHEY, Nicholas J., Culver City, CA 90232 (US)
(74) Representative: Wichmann, Hendrik
(86) International application number: PCT/US2017/060872
(87) International publication number: WO 2018/093658

(56) References cited:
- WO-A1-2013/062505
- WO-A1-2014/083555
- MATHEW J. GARNETT ET AL: "Systematic identification of genomic markers of drug sensitivity in cancer cells", NATURE, vol. 483, no. 7391, 28 March 2012 (2012-03-28), pages 570-575, XP055186003, ISSN: 0028-0836, DOI: 10.1038/nature11005
- YAHYA TAMIMI ISHITA GUPTA ET AL: "Micrometastatic Circulating Tumor cells; A Challenge for an Early Detection and Better Survival Rates", JOURNAL OF CARCINOGENESIS & MUTAGENESIS, vol. 06, no. 04, 1 January 2015 (2015-01-01), XP055447989, DOI: 10.4172/2157-2518.1000229

## Description

### Field of the Invention

The field of the invention is systems and methods of validating predicted drug responses made based on genomic and/or omics information.

### Background of the Invention

The background description includes information that may be useful in understanding the present invention. It is not an admission that any of the information provided herein is prior art or relevant to the presently claimed invention, or that any publication specifically or implicitly referenced is prior art.

Various systems and methods of computational modeling of pathways for predicting anticancer drug success in anticancer therapy are known in the art. For example, Pathway Recognition Algorithm using Data Integration on Genomic Models (PARADIGM) (Vaske et al., US20120041683 and Vaske et al., US20120158391) and Drug Intervention Response Predictions with PARADIGM (DIRPP) (Brubaker et al, 2014, Pac Symp Biocomput.: 125-135 and Vaske CJ, et al. 2010, Bioinf. 26:i237-i247) are bioinformatics systems that use omics data to calculate signaling pathway usage in a cell. PARADIGM is also designed to assess the probability of a drug being effective in a particular situation. DIRPP represents one approach to employing PARADIGM for the specific task of *in silico* drug sensitivity prediction. This is a very active area of current research, and other groups have published other approaches to probabilistic predictions of anticancer drug sensitivities. For example, Costello et al., 2014, Nature Biotechnology, 32(12): 1202-1212 reported a study comparing the strength of different methods for predicting the drug sensitivities of breast cancer cell lines as employed by multiple research groups.

Analytic and predictive omic analysis methods, such as PARADIGM, are employed to label a cancer cell type that is obtained from a subject, e.g., an individual cancer patient, as more or less probable to be sensitive to a given anticancer therapy, and to identify the genetic parameters in the cancer cell that may be helpful for determining probable drug response by a specific cancer. However, it should be clear that these methods are probabilistic, and are not validated by actual real world clinical data.

Thus, there remains a need in the art to further improve methods of optimizing anticancer therapies for individual subjects and cancer types, by validating the previous probabilistic methods with clinical data regarding the response of subject cancer cells to specific anticancer therapeutic methods and/or agents.

### Summary of The Invention

Accordingly, the invention provides a method of validating a predicted pathway activity of a pathway in a solid tumor of a subject, with a digital computer, the method comprising:
a) obtaining a tumor associated sample from the subject;
b) obtaining omics data from the tumor associated sample obtained in (a);
c) applying the omics data obtained in (b) to a digital computer programmed with a pathway analysis engine configured to generate predicted tumor cell pathway anticancer activities *in silico,* to provide a prediction of tumor cell sensitivity to one or more pharmaceutically active anticancer compounds effective for treating the subject's tumor;
d) obtaining enriched viable tumor cells from the subject, and interrogating the enriched viable tumor cells with at least one pharmaceutically active compound known to interact with a pathway element of one or more of the tumor cell pathways predicted by (c), to measure anticancer activity of the at least one pharmaceutically active compound with respect to the subject's enriched viable tumor cells; wherein the enriched viable tumor cells are at least one of the following: (i) circulating tumor cells (CTCs) isolated from the subject's blood, and (ii) tumor cells isolated from the subject and engrafted into an irradiated mouse.

The method further includes:
e) generating a validation metric by comparing the resulting anticancer activity of the at least one pharmaceutically active compound, to the tumor cell pathway anticancer activity predicted by (c); and
f) presenting the validation metric for consideration of a treatment plan targeting the solid tumor and as a function of the at least one pharmaceutically active compound.

In one embodiment the tumor associated sample from the subject is one or more of:
(a) a biopsy sample of a tumor that is extracted from the subject,
(b) enriched circulating tumor cells (CTCs) extracted from blood or body fluids of the subject, and
(c) RNA, DNA or cell-free RNA molecules shed by the subject's tumor that are isolated from the subject's blood or body fluids.

In a further embodiment, the predicted pathway activity is generated by the pathway analysis engine according to a probabilistic pathway model, e.g., a factor graph model, a Pathway Recognition Algorithm Using Data Integration on Genomic Models (PARADIGM) model and/or a Drug Intervention Response Predictions with PARADIGM (DIRPP) model.

For example, the predicted pathway activity comprises a constitutively active pathway, an up-regulated pathway, a down-regulated pathway and/or an interrupted pathway. In addition, the pathway element of step (c) comprises at least one of a DNA, an RNA, and/or a protein.

In a further example, the enriched viable tumor cells are at least one of the following:
(a) circulating tumor cells (CTCs) isolated from the subject's blood, and
(b) tumor cells isolated from the subject and engrafted into an irradiated mouse.

The step of obtaining enriched CTCs from the blood or body fluids of a subject includes subjecting the subject's blood or body fluid to one or more of dielectrophoresis, affinity separation, mass separation, FACS, LCI, and/or microfluidic cell sorting. The sorting can be conducted according to art-known methods and can include preferentially selecting CTCs that express a particular surface marker. Tumor cells isolated from the subject can also be obtained, for example, from a surgical biopsy sample.

Once the enriched viable tumor cells are obtained, the enriched viable tumor cells may be employed to generate a tumor model located external to the subject. Any art-known tumor model can be employed or adapted for the purpose. For example, the tumor model comprises an *in vitro* tissue culture model, an *in vivo* zebra fish model, an *in vivo* mouse model and/or an *in vitro* cell culture model. In certain embodiments, the tumor model is one or more of a 3D artificial tumor, a cell culture, and/or an HLA surrogate culture. In certain embodiments, the pharmaceutically active compound is a kinase inhibitor, an antibody, and/or a DNA repair inhibitor.

In certain other embodiments, the step of interrogating the enriched viable tumor cells includes exposing the enriched viable tumor cells to at least a second pharmaceutically active compound known to interact with the pathway element. The method can include, for example, interrogating the enriched viable tumor cells with the first and second pharmaceutically active compounds independently, and/or, in combination, with each other.

In the inventive method, the resulting anticancer activity is measured with any art-known assay, including, an ELISA assay and/or any other physiological parameter that is quantified after exposing the enriched viable tumor cells to the at least one pharmaceutically active compound. Other methods of measuring the physiological parameter, include, for example, one or more of a chemical parameter, a metabolic parameter, a proliferation parameter, a density parameter, and/or a viability parameter, such as by LCI, Mass Spectrometry and/or FISH, FACS, TS-phase imaging.

The method further contemplates a step of inferring a predicted sensitivity of a second pathway element to the pharmaceutically active compound, e.g., including an increased or a decreased sensitivity relative to an unmodified pathway model. In certain embodiments, the inventive method also includes correlating the predicted sensitivity with a predicted treatment outcome. In certain embodiments, the predicted pathway activity model is associated with at least one cancer cell surface marker.

Optionally the inventive method includes a step of comparing a control group activity to the predicted pathway activity. Preferably, the generation of the validation metric is a function of the control group activity comparison.

In addition, the validation metric can include a single value metric, e.g., a scalar value metric, a score, a multi-value metric, e.g., the multi-value metric represents a mean of multiple values, and/or calculated as a function of the predicted sensitivity. In a further embodiment, the validation metric includes a recommendation, and/or a predicted outcome.

Unless otherwise indicated, the terms listed below will be used and are intended to be defined as stated, unless otherwise indicated. Definitions for other terms can occur throughout the specification. It is intended that all singular terms also encompass the plural, active tense and past tense forms of a term, unless otherwise indicated.

As understood in the art, the terms "tumor" and "cancer" are overlapping terms. A "tumor" is broadly considered to be a mass or growth found in an organism. A tumor cell is a cell derived from such a mass. A tumor can be benign or cancerous. A cancerous tumor, or "cancer" is a tissue growth that can spread out of control and invade other tissues, or in the case of blood cancers, overwhelm the circulatory system and/or seed cancers elsewhere in the body. A cancer cell is a cell derived from a cancer. For purposes of the invention, the terms "tumor cell" and "cancer cell" are used interchangeably, with the understanding that both refer to mammalian cells found in tumors or cancers or derived from and cultured from tumors or cancers, and that replicate abnormally, without the limits exhibited by differentiated mammalian cells.

It should also be understood that singular forms such as "a," "an," and "the" are used throughout this application for convenience, however, except where context or an explicit statement indicates otherwise, the singular forms are intended to include the plural.

All numerical ranges should be understood to include each and every numerical point within the numerical range, and should be interpreted as reciting each and every numerical point individually. The endpoints of all ranges directed to the same component or property are inclusive, and intended to be independently combinable.

As used herein, the term "about" means within 10% of the reported numerical value, or preferably within 5% of the reported numerical value.

Various objects, features, aspects and advantages of the inventive subject matter will become more apparent from the following description of the drawing and from the detailed description of the invention.

### Brief Description of the Drawings

**FIG. 1** is a schematic copied from US20120158391, in order to illustrate one aspect of the state of the art in PARADIGM dynamic pathway map analysis.
**FIG. 2** illustrates one exemplary operation of the invention. In Fig. 2, "EVTCs" indicates enriched viable tumor cells, and "TAS" indicates a tumor associated sample.

### Detailed Description of the Invention

The invention provides methods for validating predicted drug sensitivities of cancer cells present in individual subjects. In particular, the invention provides methods for validating a predicted pathway activity of an anticancer drug sensitive pathway in a solid tumor of a subject. The method includes:
a) obtaining a tumor associated sample from the subject;
b) obtaining omics data from the tumor associated sample obtained in (a);
c) applying the omics data obtained in (b) to a pathway analysis engine configured to generate predicted tumor cell pathway activities *in silico,* to provide a prediction of one or more pharmaceutically active anticancer compounds effective for treating the subject's tumor;
d) obtaining enriched viable tumor cellsfrom the subject's blood, preferably before treatment with the one or more pharmaceutically active anticancer compounds, and interrogating the enriched viable tumor cells with at least one pharmaceutically active compound known to interact with a pathway element of one or more of the pathways predicted by (c), to measure anticancer activity of the at least one pharmaceutically active compound with respect to the subject's viable tumor cells.

The method further includes:
e) generating a validation metric by comparing the resulting activity of the at least one pharmaceutically active compound, to the tumor cell pathway activity predicted by (c); and
f) presenting the validation metric for consideration of a treatment plan targeting the solid tumor and as a function of the at least one pharmaceutically active compound.

### Overview of the Inventive Process

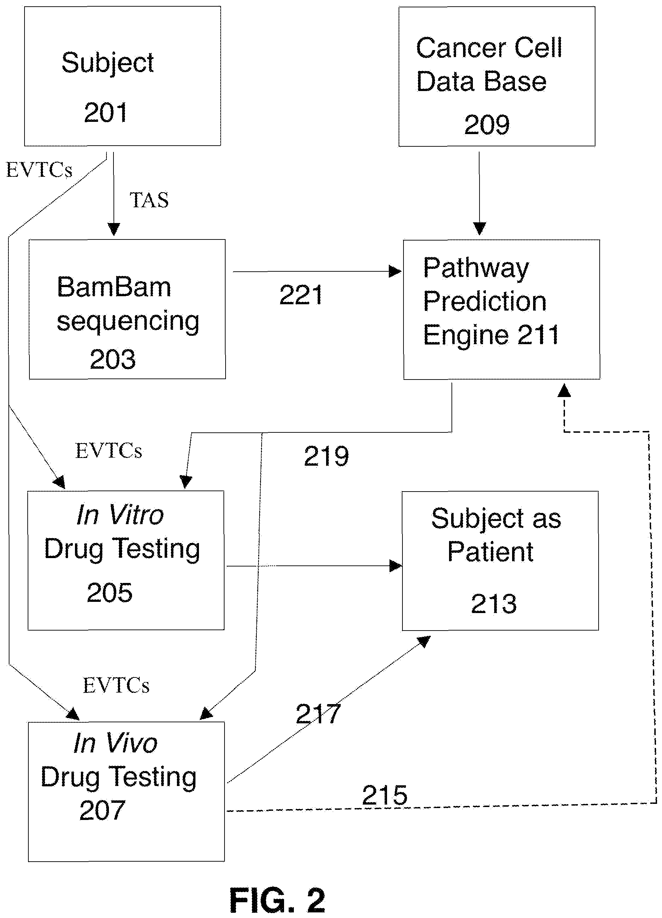

With reference to Fig. 2, the inventive process includes obtaining a sample from a subject (201). The sample can be, for instance, a biopsy sample of a suspected tumor, or a blood sample suspected to include DNA, RNA or circulating tumor cells that are associated with a tumor in the subject. In one embodiment, if the sample is a biopsy sample, and the sample is confirmed to include malignant cells, the cells are then isolated from the biopsy sample to obtain enriched viable tumor cells ("EVTCs" in Fig 2). Alternatively, instead of a biopsy source, the enriched viable tumor cells are CTCs. The enriched viable tumor cells are grown *in vitro* and/or *in vivo* environments and employed for validation testing.

In another embodiment, a tumor associated sample ("TAS" in Fig. 2), including DNA, RNA and/or proteins extracted from tumor cells from the subject, or extracted from blood or body fluids of the subject, is subjected to detailed analysis, such as BamBam analysis (203). Other types of data analysis at 203 includes genomics data (e.g., data from whole genome sequencing, whole exome sequencing, partial sequencing, gene copy numbers, allele specific information, etc.), transcriptomics data (e.g., RNAseq data, alternative splice data, etc.), proteomics data (protein activity data, quantitative protein data, e.g., provided by MS or SRM-MS methods, etc.), metabolomics data (energy load, NAD/NADH ratio, quantitative data on metabolites, etc.), immunomics date (e.g., cytokine status, chemokine status, checkpoint inhibition status, etc), etc. The omics data and/or other data characterizing the cancer cells (221) is then imported into a pathway analysis engine (211). The pathway analysis engine (211) may also import data from a cancer cell data base (209)..

The output (219) of the pathway analysis engine informs both *in vitro* (205) and *in vivo* (207) testing of enriched viable tumor cells obtained from the subject. The enriched viable tumor cells obtained from the subject are exposed to clinically relevant concentrations and durations of anticancer drugs, that are identified by the pathway prediction engine as having a useful probability for successful treatment of the subject's cancer.

The results (217) of *in vitro* (205) and *in vivo* (207) testing of the CTCs informs clinical decisions for treating the subject with one or more of the anticancer drugs that is validated as active against the subject's cancer (213). The quantitative results (215) are compared to the results provided by the pathway prediction engine to generate a validation metric, e.g., a numerical description of the difference between the predicted and validated results.

Optionally, the clinical therapy of the subject is monitored, and if there are clinical indications that the cancer has become resistant to the selected anticancer pharmaceutical or pharmaceuticals, the process may be repeated to further optimize the anticancer pharmaceutical regimen. For example, further biopsy samples of any re-grown tumor are obtained, genomic analysis is conducted, predictive pathway analysis is conducted on the obtained omic data, and the subject's enriched viable tumor cells are again subjected to validation of predicted anticancer pharmaceutical(s).

Alternatively, in the event of resistance to the selected anticancer pharmaceutical regimen, a further biopsy is not conducted, but a tumor associated sample , for example, from circulating tumor cells, or from circulating RNA or DNA, are taken from the subject, as indicated by the clinical response, to validate, modify and/or optimize the original anticancer regimen.

### Obtaining Tumor Cells from Biopsy Sample

Tumor cells can be enriched or isolated from a biopsy sample, using art-known methods, such as the Cancer Isolation Kit, sold by Affymetrix, USB (a subsidiary of ThermoFisher Scientific).

### Genomic/Omic Analysis

In one aspect, genomic analysis of the tumor associated sample obtained from the subject is conducted to obtain a set of omics data and/or to determine differences between the tumor and healthy cells of the same subject. The omic analysis can be conducted by any art-known methods, including, for example, the methods of Sanborn et al., US20120059670 and/or US20120066001. As described in detail by US20120059670 and/or US20120066001, the method includes, for example, sequencing the genomic DNA of a cancer and the genomic DNA of normal (germline) cells, and comparing results sequentially, by summarizing data at every genomic position for both cancer cells and normal cells and then combining the results for analysis.

The output of a gene sequence machine is known as a "BAM" file, and is extremely large (gigabyte to terabyte scale). To avoid the cost in time and memory needed to manipulate two different BAM files in a single central processing unit, the BamBam method is preferably employed. According to US20120059670, the "BamBam method reads from two files at the same time, constantly keeping each BAM file in synchrony with the other and piling up the genomic reads that overlap every common genomic location between the two files. For each pair of pileups, BamBam runs a series of analyses listed above before discarding the pileups and moving to the next common genomic location. By processing these massive BAM files with this method, the computer's RAM usage is minimal and processing speed is limited primarily by the speed that the file system can read the two files." Thus, a file showing only the differences is produced.

### Predicted Pathway Activity From a Pathway Analysis Engine (PARADIGM)

The above-obtained genomic and/or other omic data obtained from the subject's biopsy cancer cells is then utilized in a pathway analysis engine to generate a predicted pathway activities in the tumor, which will enable an informed choice of drug(s) for treatment.

Pathway analysis draws upon one or more databases of individual cancer cell lines. A useful database will include, for example, cancer type, genomic sequence data (omic data), including gene expression and copy number data, and anticancer drug sensitivity data for each included cancer cell line. Databases include, for example, the cancer genome project or CGP, by Garnett MJ, et al., 2012, Nature, 483(7391):570-5, the cancer cell line encyclopedia or CCLE, by Barretina J, et al., Nature, 483(7391): 603-7, the Genomics of Drug Sensitivity in Cancer (GDSC) by Yang et al., 2014 Nucleic acids research 2013;41;Database issue; D955-61 PUBMED: 23180760; PMC: 3531057; DOI: 10.1093/nar/gks1111 (the GDSC is also available in updated form via web links maintained by the Wellcome Trust, Sanger Institute (at present, the URL is www dot cancerrxgene dot org).

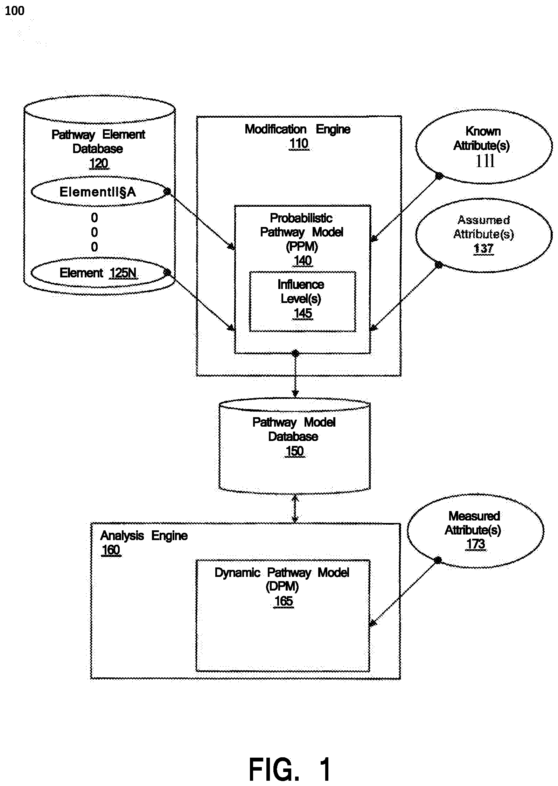

One embodiment of a PARADIGM pathway analysis is exemplified by US20120158391. **FIG. 1****,** copied from US20120158391 herein for clarity, illustrating pathway analysis ecosystem 100. As explained by US20120158391 with reference to **FIG. 1****:**
Ecosystem 100 can include pathway element database 120 preferably storing a plurality of pathway elements 125A through 125N, collectively referred to as pathway elements 125. Each of pathway elements 125 can be characterized by its involvement with one or more pathways. Elements 125 can be considered separately manageable data objects comprising one or more properties or values describing the characteristics of the element. In some embodiments, elements 125 can be considered an n-tuple of properties or values, where each property member of an element 125 tuple can be compared, analyzed, contrasted, or otherwise evaluated against other property members in other element tuples.
Modification engine 110 communicatively couples with pathway element database 120, possibly over a network link (e.g., LAN, WAN, Internet, VPN, etc.). In some embodiments, pathway element database 120 could be local to modification engine 110, while in other embodiments, pathway element database 120 could be remote from modification engine 110. For example, pathway element database 120 could be accessed via the National Lambda Rail (see URL www.nlr.net) or the Internet. Further, modification engine 110, or ecosystem 100 for that matter, can be accessed by users over the network, possibly in exchange for a fee.
Modification engine 110 obtains one or more of elements 125 from pathway element database 120 for analysis. Preferably, modification engine 110 associates at least one of elements 125 (e.g., elements 125A) with at least one a priori known attribute 133. Further, modification 110 also associates another element, element 125N for example, with assumed attribute 137. In some embodiments, modification engine 110 can make the associations automatically based on inference rules, programmatic instructions, or other techniques. For example, known attributes 137 could be obtained from known research while assumed attributes 137 could be mapped out according to an attribute parameterized space where modification engine 110 serially, or in parallel, walks through the assumed attribute space. In other embodiments, a user can manually associate attributes 133 or 137 as desired through one or more user interfaces (not shown), possibly operating through an HTTP server or other suitable interfacing technology. Modification engine 110 further cross-correlates pathway elements 125 for one or more pathways using known attributes 133 and assumed attributes 137. Further, modification engine 110 assigns one or more influence levels 145 to elements 125. Through cross-correlation and assignment of influence levels 145, modification engine 110 constructs probabilistic pathway model 140 outlining how pathways might be influenced by assumed attributes 137 or other factors.
In some embodiments, probabilistic pathway model 140 can be stored within pathway model database 150 for archival purposes, or for analysis as indicated. As with elements 125, probabilistic pathway model 140 can also be stored as a distinct manageable data object having properties or values describing the characteristics of the model, possibly as an n-tuple. Models 145, or even elements 125, can be stored according to any desirable schema. Example suitable database that can be used to construct element database 120 or model database 140 include MySQL, PostgreSQL, Oracle, or other suitable databases. In some embodiments, the data objects (e.g., elements 125, probabilistic pathway model 145, etc.) can be multiply indexed via their properties or values in a manner allowing easy searching or retrieval.
Ecosystem 100 preferably includes analysis engine 160 configured to further analyze probabilistic pathway model 150 with respect to actual data. In the example shown, analysis engine 160 obtains probabilistic pathway model 150, possibly under direction of a user or researcher, to derive dynamic pathway model 165. Preferably, dynamic pathway model 160 is derived by comparing one or more measured attributes 173 from a patient sample with the attributes associated with probabilistic pathway model 140. Thus, analysis engine 160 seeks to modify, update, correct, or otherwise validate probabilistic pathway model 140 to form dynamic pathway model 165. Once complete, dynamic pathway model 165 can be stored within a model database. In more preferred embodiments, analysis engine 160 can configure one or more output devices (e.g., a display, a printer, a web server, etc.) to present dynamic pathway model 165.

In certain embodiments, the predicted pathway activity comprises a constitutively active pathway, an up-regulated pathway, a down-regulated pathway and/or an interrupted pathway. In addition, the pathway element of step (c) comprises at least one of a DNA, an RNA, and/ or a protein.

According to the invention, a "constitutively active pathway" is a pathway that is constantly active regardless of environmental conditions or physiological demand such as the concentration of a substrate or product. Such up-regulation is typically due to a mutation in a protein involved in signaling. For example, certain point mutations in the extracellular domain of EGFR result in constitutively active EGFR proteins that signal in the absence of appropriate EGFR ligands. Similar effects have been reported for Notch signaling in solid tumors by Ranganathan et al., 2011, Nature Reviews Cancer 11: 338-351; doi:10.1038/nrc3035. It should be appreciated that such constitutively active pathways may no longer be subject to regulatory mechanisms otherwise present, and that such pathways may contain pathway elements that can be affected by one or more drugs.

According to the invention, an "up-regulated pathway" is a pathway that is at least temporarily activated relative to a 'normal' state. Such up-regulation may be due to increased quantity of pathway elements and/or ligands to activating receptors. For example, Fascin expression is up-regulated in certain cancer cells (relative to non-cancer cells) in the presence of Fas signaling-activated signal transducers and activators of transcription 3 (STAT3), Al-Alwan et al., 2011, PloS ONE 6(11) e27339; Carpenter et al., 2014, Cancers, 6: 897-925; doi: 10.3390/ cancers6020897. Similarly, it should be appreciated that such up-regulated pathways may no longer be subject to regulatory mechanisms otherwise present, and that such pathways may contain pathway elements that can be affected by one or more drugs.

According to the invention, a "down-regulated pathway" is a pathway wherein the concentration of the components are decreased in response to external stimuli. Thus, a down-regulated pathway is a pathway that is at least temporarily less active relative to a 'normal' state. Such down-regulation may be due to decreased quantity of pathway elements and/or suppressive regulatory ligands to receptors. For example, nuclear receptor binding protein 1 (NRBP1) is down-regulated in breast cancer. Wei et al., 2015, OncoTargets and Therapy 8: 3721-3730. It should be appreciated that such down-regulated pathways may no longer contribute to processes critical for normal cell function, and that such pathways may contain pathway elements that can be stimulated by one or more drugs to so reactivate the pathway.

According to the invention, an "interrupted pathway" is a pathway wherein a single or multiple components has been interfered or inactivated by an external cause. For example, it is a pathway in which at least one pathway element is missing such that signal flow through the pathway is interrupted.

According to the invention, a "pathway element" or pathway component is a chemical species converted in a series of linked chemical reactions in a cellular metabolic or genetic regulatory pathway. In a PARADIGM analysis, as described in WO2013062505 and US 20120158391, these include, for example, a pathway element that is an element in a factor graph (an 'entity' in the probabilistic model). In a simple form, US20120158391 describes elements in Fig.2 of that publication, and associated paragraphs [0010] through [0016], and particularly paragraph [0010] exemplifies a pathway element as including, for example "pathway elements such as a protein selected from the group consisting of a receptor, a hormone binding protein, a kinase, a transcription factor, a methylase, a histone acetylase, and a histone deacetylase or a nucleic acid is selected from the group consisting of a genomic regulatory sequence, a regulatory RNA, and a trans-activating sequence."

Pathway analysis results in a predicted assessment of the probability that one or more anticancer drugs will be effective in treating the cancer, represented by the tumor associated sample and/or viable tumor cells, or other cancer cells or cancer tissue that is obtained from the subj ect.

### Obtaining Enriched Circulating Tumor Cells (CTCs) or Biopsy Tumor Cells

The step of obtaining the enriched CTCs from the blood or body fluids of a subject includes subjecting the subject's blood or body fluid to one or more of dielectrophoresis, affinity separation, mass separation, fluorescence activated cell sorting (FACS), live cell interferometry (LCI) and/or microfluidic cell sorting. The sorting can be conducted according to art-known methods and can include preferentially selecting CTCs that express a particular surface marker. Circulating tumor cells can be enriched from a subject's circulating blood or body fluids by any suitable art-known methods. For example, CTCs can be enriched from a first buffy coat layer from a blood sample, by density gradient centrifugation, as described by Thomas, in US20160223557. CTCs can also be enriched from whole blood, as described by Fuchs et al. in US8921102 by capturing CTCs in a two-dimensional array microfluidic device tagged with capture moieties such as, for example, EpCAM, E-Cadherin, Mucin-I, Cytokeratin 8, EGFR, and leukocyte associated receptor (LAR).

Once the viable tumor cells are obtained from the subject, the cancer cells are employed to generate a tumor model located external to the subject. Any art-known tumor model can be employed or adapted for the purpose. For example, the tumor model comprises an *in vitro* tissue culture model, an *in vivo* zebra fish model, an *in vivo* mouse model and/or an *in vitro* cell culture model. In certain embodiments, the tumor model is one or more of a 3D artificial tumor, a cell culture, and/or an HLA surrogate culture. In certain other embodiments, the pharmaceutically active compound is a kinase inhibitor, an antibody, and/or a DNA repair inhibitor. In still further embodiments, the viable tumor cells (e.g., circulating tumor cells or from secondary biopsy or surgery) may also be directly exposed to at least one pharmaceutically active compound known to interact with a pathway element of the pathway to obtain a resulting activity. Such direct exposure will typically be performed in vitro using defined media conditions, or media simulating the nutrient and gas partial pressures within a tumor.

### Interrogating Enriched Viable Tumor Cells Obtained from the Subject

Broadly, the term "interrogating" refers to any method of analyzing and measuring the properties of a composition of interest, e.g., cancer cells.

### Drug Sensitivity Validation Interrogation of Viable Tumor Cellss

For *in vitro* testing, enriched viable tumor cells from a subject can be tested for drug sensitivities by any art-known methods. For example, enriched viable tumor cells can be tested while cultured in a collagen gel substrate, e.g., according to the teachings of US5356793. For example, enriched vable tumor cells can be tested while cultured on layers of liver and bone marrow cells, as taught by US9389220. Viable tumor cells can also be tested while cultured according to the methods taught by US5455161 and/or US6448030.

In addition, the enriched viable tumor cells are also contemplated to be interrogated in a suitable art-known 3D tissue culture system, e.g., as described by US20150065359, or in the culture systems taught by co-owned US20160108358, published on April 21, 2016 and co-owned US20160083682, published on March 24, 2016. These tissue culture systems taught by the aforementioned patent documents include, for example, a continuous culture device comprising (a) a scaffold formed by a matrix of interconnected growth surfaces spaced at regular intervals and (b) a fluid distribution means at the inlet and the exit of the growth areas. The spacing and definition are arranged to permit directional flow through and around the growth surfaces uniformly. The fluid distribution means at the inlet and the exit of the growth areas permits an adequate flow to each growth surfaces. The fluid distribution is analyzed using computational fluid dynamics and key metabolite utilization analysis to assure that the cells are not subject to detrimental growth conditions. In more detail, the 3D system optionally includes the following components.

A scaffold as taught by co-owned US20160108358 and/or co-owned US20160083682 comprises a 3D lattice-shaped growth surfaces forming a matrix of interconnected orthogonal growth surfaces spaced along and parallel to x, y, and z Cartesian axes, the growth surfaces forming a matrix of cubic open spaces arranged parallel to the x, y, and z Cartesian axes. The scaffold can be fabricated from any art-known biocompatible materials, e.g., polycaprolacton, polyethylene oxide - terephthalate, polyamide, poly-L-lactic acid, polyglycolic acid, collagen, fibronectin, hydroxyapatite, and the like.

In a practical preferred embodiment, the culture device further comprises an aseptically sealed housing that can be disassembled at the completion of the culture period. Said aseptic housing can include a sealed removable cover, an inlet distribution means, an optional exit distribution means, and the necessary support means required to locate and secure the growth surfaces in the culture device.

An inlet fluid distribution device connected to an input side of the scaffold and having a common fluid inlet that is fluidly coupled with a plurality of inlet distribution conduits through which input fluid is delivered to and distributed through the open spaces of the scaffold.

An outlet fluid collection device connected to an output side of the scaffold and having a plurality of outlet conduits that are fluidly coupled with a common fluid outlet that collects output fluid from the open spaces of the scaffold.

A housing coupled to the inlet fluid distribution device and the outlet fluid collection device, and that houses the scaffold, inlet fluid distribution device, and outlet fluid collection device.

Wherein the housing, scaffold, inlet fluid distribution device, and outlet fluid collection device comprise a single work piece.

The enriched viable tumor cells are contemplated to be cultured employing art-known methods, including culturing in the above-described apparatus and methods, or analogous equipment and methods for culturing tumor cells in a 3D tissue system. In such a system, inoculated viable tumor cells form 3D *in vitro* model tumors, and a drug or drugs predicted to be effective against the viable tumor cells by pathway analysis are validated in a range of concentrations, durations and combinations. Optimal anticancer drugs or drug combinations, doses and/or durations, validated by the *in vitro* testing are then considered as the basis for clinical treatment of the subject from which the cancer cells are obtained.

For *in vivo* testing, viable tumor cells can be tested for drug sensitivities, i.e., sensitivity to pharmaceutically active anticancer compounds, in any suitable animal xenograft model, including an *in vivo* zebra fish model and/or an *in vivo* mouse model. For example, viable tumor cells can be transplanted into immune compromised zebra fish as described by US20160166713, or into an immune deficient nude mouse model, for example, as described by US6107540. Other suitable non-human animal models known to the art are optionally employed, including, e.g., rat, hamster, or other rodent; rabbit, pig, guinea pig, or dog as described, for example, by US20140047570.

Obtained viable tumor cells are injected into the muscle, viscera, and/or other tissues of the test animal, and tumor growth is followed by art-known methods. Once the xenografted tumor mass reaches a measurable size, a drug or drugs predicted to be effective against the enriched cancer cells by pathway analysis are validated in a range of concentrations, durations and combinations. Optimal validated anticancer drugs or drug combinations, doses and/or durations, are then considered as the basis for clinical treatment of the subject from which the enriched cancer cells are obtained.

In certain other embodiments, the step of interrogating the viable tumor cells includes exposing the viable tumor cells, or artificial tumor developed from those viable tumor cells, to at least a second pharmaceutically active compound known to interact with the pathway element. The method can include, for example, interrogating the cultured viable tumor cells with the first and second pharmaceutically active compounds independently, and/or, in combination, with each other.

In the inventive method, the resulting activity is measured with any art-known assay, including, an ELISA assay or any other measurable physiological parameter that is quantified after exposing the cancer cells to the at least one pharmaceutically active compound. Other methods of measuring the physiological parameter, include, for example, one or more of a chemical parameter, a metabolic parameter, a proliferation parameter, a density parameter, and/or a viability parameter, such as by LCI, Mass Spectrometry and/or FISH, FACS, TS-phase imaging.

The method further contemplates a step of inferring a predicted sensitivity of a second pathway element to the pharmaceutically active compound, e.g., including an increased or a decreased sensitivity relative to an unmodified pathway model. In certain embodiments, the inventive method also includes correlating the predicted sensitivity with a predicted treatment outcome. In certain embodiments, the predicted pathway activity model is associated with at least one cancer cell surface marker.

Optionally the inventive method includes a step of comparing a control group activity to the predicted pathway activity. Preferably, the generation of the validation metric is a function of the control group activity comparison.

In addition, the validation metric can include a single value metric, e.g., a scalar value metric, a score, a multi-value metric, e.g., the multi-value metric represents a mean of multiple values, and/or calculated as a function of the predicted sensitivity. In a further embodiment, the validation metric includes a recommendation, and/or a predicted outcome.

## Claims

1. A method of validating a predicted pathway activity of a pathway in a solid tumor of a subject, with a digital computer, the method comprising:
a) obtaining omics data from a tumor associated sample obtained from the subject;
b) applying the omics data obtained in (a) to a digital computer programmed with a pathway analysis engine configured to generate predicted tumor cell pathway activities *in silico,* to provide a prediction of tumor cell sensitivity to one or more pharmaceutically active anticancer compounds effective for treating the subject's tumor;
c) interrogating enriched viable tumor cells obtained from the subject with said at least one pharmaceutically active compound known to interact with a pathway element of one or more of the tumor cell pathways predicted by (b), to measure anticancer activity of the at least one pharmaceutically active compound with respect to the subject's enriched viable tumor cells; wherein the enriched viable tumor cells are at least one of the following:
(i) circulating tumor cells (CTCs) isolated from the subject's blood, and
(ii) tumor cells isolated from the subject and engrafted into an irradiated mouse.

2. The method of claim 1, further comprising the steps of,
d) generating a validation metric by comparing the measured anticancer activity of the at least one pharmaceutically active compound, to the tumor cell pathway activity predicted by step (b); and
e) presenting the validation metric for consideration of a treatment plan targeting the solid tumor and as a function of the at least one pharmaceutically active compound.

3. The method of claim 1, wherein tumor associated sample from the subject is selected from the group consisting of:
(a) a biopsy sample of a tumor that is extracted from the subject,
(b) enriched circulating tumor cells (CTCs) extracted from blood or body fluids of the subject, and
(c) RNA, DNA or cell-free RNA molecules shed by the subject's tumor that are isolated from the subject's blood or body fluids.

4. The method of claim 1, wherein the predicted pathway activity is generated by the pathway analysis engine according to a probabilistic pathway model; in particular wherein the probabilistic pathway model is selected from the group consisting of a factor graph model, a PARADIGM model or a Drug Intervention Response Predictions with PARADIGM (DIRPP) model.

5. The method of claim 1, wherein the predicted pathway activity comprises a pathway selected from the group consisting of a constitutively active pathway, an up-regulated pathway, and a down-regulated pathway and an interrupted pathway.

6. The method of claim 1, wherein the pathway element comprises at least one of a DNA, an RNA, and a protein.

7. The method of claim 1, wherein the enriched viable tumor cells have been obtained by a procedure comprising one or more of dielectrophoresis, affinity separation, mass separation, FACS, LCI, and microfluidic cell sorting; optionally, wherein said procedure further comprises preferentially selecting viable tumor cells that express a specific surface marker.

8. The method of claim 1, further comprising a step of generating, external to the patient, a tumor model comprising the enriched viable tumor cells; in particular wherein the tumor model comprises a model selected from the group consisting of an *in vitro* tissue culture model, an *in vitro* cell culture model, a 3D artificial tumor, a tumor cell culture, and an HLA surrogate culture.

9. The method of claim 1, wherein the resulting anticancer activity is measured by quantifying a physiological parameter after exposing the enriched viable tumor cells to the at least one pharmaceutically active compound, in particular wherein the physiological parameter is measured by one or more of a chemical parameter, a metabolic parameter, a proliferation parameter, a density parameter, a viability parameter;
or wherein the resulting anticancer activity is measured by one or more of LCI, Mass Spectrometry, FISH, FACS, TS-phase imaging or ELISA assay.

10. The method of claim 1, further comprising a step of modifying the predicted pathway activity model.

11. The method of claim 1, further comprising a step of inferring a predicted sensitivity of a second pathway element to the pharmaceutically active compound; in particular wherein the predicted sensitivity is an increased sensitivity relative to an unmodified pathway model or is a decreased sensitivity relative to an unmodified pathway model; or further comprising correlating the predicted sensitivity with a predicted treatment outcome.

12. The method of claim 1, further comprising a step of comparing a control group activity to the predicted pathway activity; in particular wherein the generation of the validation metric is a function of the control group activity comparison; more particularly wherein the single value metric is a scalar or a score; or wherein the multi-value metric is a mean of multiple values or is a distribution of a range of multiple values.

13. The method of claim 1, wherein the step of interrogating the enriched viable tumor cells further comprises exposing the enriched viable tumor cells to at least a second pharmaceutically active compound known to interact with the pathway element; in particular further comprising interrogating the enriched viable tumor cells with the first and second pharmaceutically active compounds independently of each other or in combination with each other.

14. The method of claim 2, wherein (i) the validation metric is calculated as a function of the predicted sensitivity; or (ii) wherein the validation metric comprises a single value metric or a multi-value metric; or (iii) wherein the validation metric comprises a recommendation or a predicted outcome; or (iv) wherein the predicted pathway activity model is associated with at least one cancer cell surface marker.

## Patentansprüche

1. Verfahren zur Validierung einer vorhergesagten Signalwegaktivität eines Signalwegs in einem soliden Tumor eines Patienten mit einem digitalen Computer, wobei das Verfahren Folgendes umfasst:
a) Ermitteln von Omics-Daten aus einer Tumor-assoziierten Probe, die vom Patienten gewonnen wurde;
b) Anwenden der in (a) erhaltenen Omics-Daten auf einen digitalen Computer, der mit einer Signalweganalysemaschine programmiert ist, die so konfiguriert ist, dass sie prognostizierte Tumorzellsignalwegaktivitäten *in silico* generiert, um eine Prognose der für die Behandlung des Tumors des Patienten wirksamen Tumorzellempfindlichkeit gegenüber einer oder mehreren pharmazeutisch aktiven Antikrebsverbindungen zu liefern;
c) Untersuchen angereicherter lebensfähiger vom Patienten gewonnener Tumorzellen mit der mindestens einen pharmazeutisch aktiven Verbindung, die bekanntermaßen mit einem Signalwegelement eines oder mehrerer der durch (b) prognostizierten Tumorzellsignalwege interagiert/interagieren, um die Antikrebsaktivität der mindestens einen pharmazeutisch aktive Verbindung gegenüber den angereicherten lebensfähigen Tumorzellen des Patienten zu messen;
wobei die angereicherten lebensfähigen Tumorzellen mindestens eine der folgenden sind:
(i) aus dem Blut des Patienten isolierte zirkulierende Tumorzellen (CTCs), und
(ii) Tumorzellen, die aus dem Subjekt isoliert und in eine bestrahlte Maus transplantiert wurden.

2. Verfahren nach Anspruch 1, ferner umfassend die Schritte:
d) Generieren einer Validierungsmetrik durch Vergleichen der gemessenen Antikrebsaktivität der mindestens einen pharmazeutisch aktiven Verbindung mit der in Schritt (b) prognostizierten Tumorzellsignalwegaktivität, und
e) Vorlegen der Validierungsmetrik zur Prüfung eines Behandlungsplans gegen den soliden Tumor und als eine Funktion der mindestens einen pharmazeutisch wirksamen Verbindung.

3. Verfahren nach Anspruch 1, wobei die tumorassoziierte Probe des Patienten ausgewählt ist unter:
(a) einer Biopsieprobe eines aus dem Patienten entnommen Tumors,
(b) angereicherten zirkulierenden Tumorzellen (CTCs), die aus Blut oder Körperflüssigkeiten des Patienten entnommen sind, und
(c) RNA, DNA oder vom Tumor des Patienten ausgeschiedene zellfreie RNA-Moleküle, die aus dem Blut oder den Körperflüssigkeiten des Patienten isoliert sind.

4. Verfahren nach Anspruch 1, wobei die prognostizierte Signalwegaktivität von der Signalweganalysemaschine gemäß einem probabilistischen Pfadmodell generiert wird; insbesondere wobei das probabilistische Pfadmodell ausgewählt ist unter einem Faktorgraphenmodell, einem PARADIGM-Modell oder einem Drug-Intervention-Response-Predictions-with-PARADIGM-(DIRPP-)Modell.

5. Verfahren nach Anspruch 1, wobei die prognostizierte Signalwegaktivität einen Signalweg umfasst, der ausgewählt ist unter einem konstitutiv aktiven Signalweg, einem hochregulierten Signalweg und einem herunterregulierten Signalweg und einem unterbrochenen Signalweg.

6. Verfahren nach Anspruch 1, wobei das Signalwegelement mindestens eines der folgenden umfasst: eine DNA, eine RNA und ein Protein.

7. Verfahren nach Anspruch 1, wobei die angereicherten lebensfähigen Tumorzellen durch ein Verfahren gewonnen wurden, das eines oder mehrere der folgenden umfasst: Dielektrophorese, Affinitätstrennung, Massentrennung, FACS, LCI und mikrofluidische Zellsortierung; wobei das Verfahren optional weiterhin ein bevorzugtes Auswählen lebensfähiger Tumorzellen umfasst, die einen spezifischen Oberflächenmarker exprimieren.

8. Verfahren nach Anspruch 1, das ferner einen Schritt des Generierens eines die angereicherten lebensfähigen Tumorzellen umfassenden Tumormodells außerhalb des Patienten umfasst; insbesondere wobei das Tumormodell ein Modell umfasst, das ausgewählt ist unter: einem *In*-*vitro*-Gewebekulturmodell, einem *In*-*vitro*-Zellkulturmodell, einem künstlichen 3D-Tumor, einer Tumorzellkultur und einer HLA-Ersatzkultur.

9. Verfahren nach Anspruch 1, wobei die resultierende Antikrebsaktivität durch Quantifizieren eines physiologischen Parameters gemessen wird, nachdem die angereicherten lebensfähigen Tumorzellen der mindestens einen pharmazeutisch aktiven Verbindung ausgesetzt wurden, insbesondere wobei der physiologische Parameter durch einen oder mehrere der folgenden gemessen wird: einen chemischen Parameter, einen Stoffwechselparameter, einen Proliferationsparameter, einen Dichteparameter, einen Lebensfähigkeitsparameter; oder wobei die resultierende Antikrebsaktivität durch einen oder mehrere der folgenden Tests gemessen wird: LCI, Massenspektrometrie, FISH, FACS, TS-Phasen-Bildgebung oder ELISA-Assay.

10. Verfahren nach Anspruch 1, das ferner einen Schritt des Modifizierens des prognostizierten Signalwegaktivitätsmodells umfasst.

11. Verfahren nach Anspruch 1, das ferner einen Schritt des Ableitens einer prognostizierten Empfindlichkeit eines zweiten Signalwegelements gegenüber der pharmazeutisch aktiven Verbindung umfasst; insbesondere wobei es sich bei der prognostizierten Empfindlichkeit um eine erhöhte Empfindlichkeit im Vergleich zu einem unmodifizierten Signalwegmodell oder um eine verringerte Empfindlichkeit im Vergleich zu einem unmodifizierten Signalwegmodell handelt; oder das ferner das Korrelieren der prognostizierten Empfindlichkeit mit einem prognostizierten Behandlungsergebnis umfassend.

12. Verfahren nach Anspruch 1, weiterhin umfassend einen Schritt des Vergleichens einer Kontrollgruppenaktivität mit der prognostizierten Signalwegaktivität; insbesondere wobei die Generierung der Validierungsmetrik eine Funktion des Aktivitätsvergleichs der Kontrollgruppe ist; insbesondere wobei die Einzelwertmetrik ein Skalar oder ein Punktwert ist; oder wobei die Mehrwertmetrik ein Mittelwert mehrerer Werte oder eine Verteilung eines Bereichs mehrerer Werte ist.

13. Verfahren nach Anspruch 1, wobei der Schritt der Untersuchung der angereicherten lebensfähigen Tumorzellen weiterhin umfasst, dass die angereicherten lebensfähigen Tumorzellen mindestens einer zweiten pharmazeutisch aktiven Verbindung ausgesetzt werden, die bekanntermaßen mit dem Signalwegelement interagiert; insbesondere ferner umfassend das Untersuchen der angereicherten lebensfähigen Tumorzellen mit den ersten und zweiten pharmazeutisch aktiven Verbindungen unabhängig voneinander oder in Kombination miteinander.

14. Verfahren nach Anspruch 2, wobei (i) die Validierungsmetrik als Funktion der prognostizierten Empfindlichkeit berechnet wird; oder (ii) wobei die Validierungsmetrik eine Einzelwertmetrik oder eine Mehrwertmetrik umfasst; oder (iii) wobei die Validierungsmetrik eine Empfehlung oder ein prognostiziertes Ergebnis umfasst; oder (iv) wobei das prognostizierte Signalwegaktivitätsmodell mit mindestens einem Krebszelloberflächenmarker assoziiert ist.

## Revendications

1. Procédé de validation d'une activité de voie prédite d'une voie dans une tumeur solide d'un sujet, avec un calculateur numérique, le procédé comprenant :
a) l'obtention de données omiques à partir d'un échantillon associé à une tumeur obtenu auprès du sujet ;
b) l'application des données omiques obtenues en (a) à un calculateur numérique programmé avec un moteur d'analyse de voie conçu pour générer des activités prédites de voie de cellule tumorale *in silico,* afin de fournir une prédiction de la sensibilité des cellules tumorales à un ou plusieurs composés anticancéreux pharmaceutiquement actifs, efficaces pour traiter la tumeur du sujet ;
c) l'interrogation de cellules tumorales viables enrichies obtenues à partir du sujet avec ledit au moins un composé pharmaceutiquement actif connu pour interagir avec un élément de voie d'une ou plusieurs des voies de cellules tumorales prédites par (b), pour mesurer l'activité anticancéreuse dudit au moins un composé pharmaceutiquement actif par rapport aux cellules tumorales viables enrichies du sujet ; les cellules tumorales viables enrichies étant au moins l'une des suivantes :
(i) des cellules tumorales circulantes (CTC) isolées du sang du sujet, et
(ii) des cellules tumorales isolées du sujet et greffées chez une souris irradiée.

2. Procédé selon la revendication 1, comprenant en outre les étapes de :
d) génération d'une métrique de validation par comparaison de l'activité anticancéreuse mesurée du ou des composés pharmaceutiquement actifs à l'activité de la voie des cellules tumorales prédite par l'étape (b) ; et
e) présentation de la métrique de validation pour la prise en compte d'un plan de traitement ciblant la tumeur solide et en fonction du ou des composés pharmaceutiquement actifs.

3. Procédé selon la revendication 1, dans lequel l'échantillon associé à une tumeur provenant du sujet est sélectionné dans le groupe constitué de :
(a) un échantillon de biopsie d'une tumeur qui est extraite du sujet,
(b) des cellules tumorales circulantes (CTC) enrichies, extraites du sang ou des fluides corporels du sujet, et
(c) des molécules d'ARN, d'ADN ou d'ARN acellulaire excrétées par la tumeur du sujet qui sont isolées du sang ou des fluides corporels du sujet.

4. Procédé selon la revendication 1, dans lequel l'activité de voie prédite est générée par le moteur d'analyse de voie selon un modèle de voie probabiliste ; en particulier, dans lequel le modèle de voie probabiliste est sélectionné dans le groupe constitué par un modèle graphique factoriel, un modèle PARADIGM ou un modèle de prédictions de la réponse à une intervention médicamenteuse avec PARADIGM (DIRPP).

5. Procédé selon la revendication 1, dans lequel l'activité de voie prédite comprend une voie sélectionnée dans le groupe constitué par une voie constitutivement active, une voie régulée positivement, et une voie régulée négativement et une voie interrompue.

6. Procédé selon la revendication 1, dans lequel l'élément de voie comprend au moins un élément parmi un ADN, un ARN et une protéine.

7. Procédé selon la revendication 1, dans lequel les cellules tumorales viables enrichies ont été obtenues par une procédure comprenant une ou plusieurs parmi la diélectrophorèse, la séparation par affinité, la séparation selon la masse, le FACS, le LCI et le tri cellulaire microfluidique ; éventuellement, dans lequel ladite procédure comprend en outre la sélection préférentielle de cellules tumorales viables qui expriment un marqueur de surface spécifique.

8. Procédé selon la revendication 1, comprenant en outre une étape de génération, externe au patient, d'un modèle de tumeur comprenant les cellules tumorales viables enrichies ; en particulier, dans lequel le modèle de tumeur comprend un modèle sélectionné dans le groupe constitué par un modèle de culture tissulaire *in vitro,* un modèle de culture cellulaire *in vitro,* une tumeur artificielle 3D, une culture de cellules tumorales et une culture de substitution HLA.

9. Procédé selon la revendication 1, dans lequel l'activité anticancéreuse résultante est mesurée par quantification d'un paramètre physiologique après exposition des cellules tumorales viables enrichies audit au moins un composé pharmaceutiquement actif, en particulier dans lequel le paramètre physiologique est mesuré par un ou plusieurs éléments parmi un paramètre chimique, un paramètre métabolique, un paramètre de prolifération, un paramètre de densité, un paramètre de viabilité ;
ou dans lequel l'activité anticancéreuse résultante est mesurée par un ou plusieurs éléments parmi la LCI, la spectrométrie de masse, la FISH, la FACS, l'imagerie en phase TS ou le dosage ELISA.

10. Procédé selon la revendication 1, comprenant en outre une étape de modification du modèle d'activité de voie prédite.

11. Procédé selon la revendication 1, comprenant en outre une étape de déduction d'une sensibilité prédite d'un deuxième élément de voie au composé pharmaceutiquement actif ; en particulier, dans lequel la sensibilité prédite est une sensibilité accrue par rapport à un modèle de voie non modifié ou est une sensibilité diminuée par rapport à un modèle de voie non modifié ; ou comprenant en outre la corrélation de la sensibilité prédite avec un résultat de traitement prédit.

12. Procédé selon la revendication 1, comprenant en outre une étape de comparaison d'une activité de groupe témoin à l'activité de voie prédite ; en particulier, dans lequel la génération de la métrique de validation est une fonction de la comparaison d'activité du groupe témoin ; plus particulièrement, dans lequel la métrique à valeur unique est un scalaire ou un score ; ou dans lequel la métrique à valeurs multiples est une moyenne de plusieurs valeurs ou est une distribution d'une plage de plusieurs valeurs.

13. Procédé selon la revendication 1, dans lequel l'étape d'interrogation des cellules tumorales viables enrichies comprend en outre l'exposition des cellules tumorales viables enrichies à au moins un deuxième composé pharmaceutiquement actif connu pour interagir avec l'élément de la voie ; comprenant en particulier, en outre, l'interrogation des cellules tumorales viables enrichies avec les premier et deuxième composés pharmaceutiquement actifs indépendamment l'un de l'autre ou en combinaison l'un avec l'autre.

14. Procédé selon la revendication 2, dans lequel (i) la métrique de validation est calculée en fonction de la sensibilité prédite ; ou (ii) dans lequel la métrique de validation comprend une métrique à valeur unique ou une métrique à valeurs multiples ; ou (iii) dans lequel la métrique de validation comprend une recommandation ou un résultat prédit ; ou (iv) dans lequel le modèle d'activité de voie prédit est associé à au moins un marqueur de surface de cellule cancéreuse.
